# EUROPEAN PATENT APPLICATION

(11) **EP 2 343 067 A1**
(43) Date of publication of application: **13.07.2011**
(21) Application number: 10006525.9
(22) Date of filing: 23.06.2010
(51) Int. Cl.: A61K 31/341, A61K 31/43, A61K 31/431, A61K 31/4439, A61K 31/545, A61K 31/546, A61K 31/65, A61K 31/7048, A61K 9/00, A61K 9/10, A61K 9/14, A61K 45/06, A61P 1/04

(54) **Pharmaceutical composition for the eradication of helicobacter pylori and preparation method thereof**

(30) Priority: 31.12.2009 TW 098146646
(71) Applicant: Synmosa Biopharma Corporation, Hsin Chu Hsien (TW); Chou, Jui-Ming, Neihu Dist Taipei City 114 (TW); Pao, Li-Heng, Zhongzheng Taipei 100 (TW)
(72) Inventor: Lin, Chih-Hui, Hwu Kuo Hsiang Hsin Chu Hsien (TW); Chou, Jui-Ming, Neihu Dist Taipei City 114 (TW); Pao, Li-Heng, Zhongzheng Dist. Taipei City 100 (TW); Chang, Chia-Wen, Neihu Dist. Taipei City 114 (TW); Lin, Chia-Yi, Xindian City Taipei County 231 (TW); Lee, An-Long, Neihu Dist. Taipei City 114 (TW)
(74) Representative: Winkler, Andreas Fritz Ernst

(57) **Abstract**

The present invention relates to a pharmaceutical composition and its preparation method for the eradication of *Helicobacter pylori* in the forms of effervescent tablet, suspension or powder. The pharmaceutical composition comprises an effective dose of β-lactam antibiotic, an effective dose of macrolide antibiotic, an effective dose of antacid such as proton pump inhibitor and H₂ blocker, and a pharmaceutical acceptable carrier. An effective dose of alkaline substance such as carbonate or bicarbonate can be added to increase the pH of the stomach when the PPI antacid is used, which can protect the degradation of acid-labile antibiotics or PPI to further increase the bioavailability of the pharmaceutical composition for the purpose of *Helicobacter pylori* eradication.

## Description

### BACKGROUND OF THE INVENTION

### 1. Field of the Invention

The present invention relates to a pharmaceutical composition for the inhibition of *Helicobacter pylori* and preparation method thereof, in particular to a pharmaceutical composition in forms of effervescent tablets, suspension or powder for the eradication of *Helicobacter pylori* and preparation method thereof.

### 2. The Prior Arts

*Helicobacter pylori* is a Gram-negative, microaerophilic rod that can inhabit in the mucous membranes lining the stomach (gastric mucos layer) of human or other animals. At least half the world's population is infected by the bacterium. Studies have shown that persistent infection caused pathological changes in upper digestive tract. It is strongly associated with chronic active gastritis, peptic ulcer, non-ulcer dyspepsia, GERD, gastric ulcer, duodenal ulcer and so on. Moreover, infection with *Helicobacter pylori* is thought to be closely associated with gastric carcinoma, gastric adenocarcinoma, maltoma. Therefore, *Helicobacter pylori* has been classified as a type 1 (definite) carcinogen.

On the other hand, various types of ulcers tend to recur frequently and lead to significantly higher risk of gastric cancer if the *Helicobacter pyloriis* not eradicated and gastric cancer maybe induced significantly in the future. For example, the recurrence rate of peptic ulcer induced by *Helicobacter pylori* is around 60%-95% if the *Helicobacter pyloriis* not eradicated, while the ulcer recurrence rate can be dropped to below 20% after eradication of *Helicobacter pylori.* Therefore, the eradication of *H+elicobacter pylori* plays a key role in treatment and prognosis of the ulcer patients. *Helicobacter pylori* can persist for decades or even the whole life of the subject unless eradicated by proper treatment.

For the eradication of *Helicobacter pylori,* a combination therapy using two antibiotics and an antacid (H₂ blocker or proton pump inhibitor [PPI]) currently, where penicillin antibiotics (such as amoxicillin and metronidazole), and macrolide antibiotics (such as clarithromycin and tetracycline) were frequently used. Antacid H₂ blocker increases intragastric pH value, while PPI suppresses the production of stomach acid. Antibiotics such as clarithromycin are more stable in mildly acidic to basic condition, and are sensitive to degradation of gastric acid. Therefore addition of antacids can increase intragastric pH to further help the anti-*Helicobacter pylorieffects.* The duration of the regimes is often 7 days or 10-14 days. The eradication rate (>85%) can be achieved if the patients had good compliance.

However, the efficacy was not as high as predicated though the triple therapy is an effective *Helicobacter pylori* eradication therapy. There are 5 possible explanations: 1. poor compliance: patients were either given 5 tablets each time and 2 times daily, which include omeprazole (20 mg twice daily), amoxicillin (500 mg two tablets each time), clarithromycin (250 mg two tablets each time) in a 7 to 14 day course for the triple therapy; or a 3-in-1 PYLERA® capsule (3 times daily) plus omeprazole (20 mg twice daily) for the PYLERA® triple therapy. The major drawbacks in both ways include keeping track of pills in large quantity each time, confusing with the number or types of medications, not easy to carry and swallowing difficulty. These complexities led to poor compliance; 2. low dosage: low dosage during treatment course also dues to incorrect taking of the prescribed medicines; 3. Instability of medicines in stomach resulted in poor absorption; 4. the physiques of the patients and drug allergy; and 5. drug resistance of *Helicobacter pylori.:* acquired resistance to metronidazole appears to be common in patients who failed to respond to previous triple therapy. Therefore, the use of metronidazole should be aware of its dosage, the period of treatment and the patient compliance. In addition, U.S. Patent No. 5,196,205 described a complicated use of bismuth together with penicillin and tetracyclin to treat *Helicobacter pylori* -induced gastritis, gastric ulcer and duodenal ulcer patients, which applied 3 tablets (one for each active ingredient) several times daily.

On the other hand, the stability of antacid such as omeprazole is a function of pH; it is rapidly degraded in acidic condition (in the stomach) while keep stable in basic condition. Therefore, an enteric coating was applied over the core to prevent the omeprazole from contacting the acidic pH conditions of the stomach. The absorption of omeprazole takes place in the intestine and inhibits the release of gastrin from the gastric mucosa the gastric acid is inhibited via circulation. Omeprazole would not inhibit gastric acid secretion in acidic condition if acts on stomach directly, and the efficacy of antibiotics to eradicate *Helicobacter pylori* would be decreased.

U.S. Patent No. 6,350,468 disclosed a double capsule where an internal capsule was put inside an external capsule. The external capsule contains bismuth subcitrate and metronidazole, and the internal capsule contains tetracyclin and omeprazole. The active ingredients can be activated at the right intervals of time and in the preestablished quantities to eradicate *Helicobacter pylori.* U.S. Patent No. 6,977,086 also described a pharmaceutical formulation comprising amoxycillin and potassium clavulanate which is lighter than the current medicines and can be produced in the forms of tablet, capsule or powder to treat bacterial infection.

In summary, the present therapy for *Helicobacter pylori* eradication has the abovementioned drawbacks. An alternative form of *Helicobacter pylori* eradication agent in improving patient compliance and is easy to swallow for children and elderly people would be beneficial in treating *Helicobacter pylori* caused upper gastrointestinal diseases and gastric cancer.

### SUMMARY OF THE INVENTION

To solve the abovementioned problem and drawbacks of *Helicobacter pylori* triple therapy, the present invention provides a pharmaceutical composition for the eradication of *Helicobacter pylori,* which comprises an effective dose of β-lactam antibiotic, an effective dose of macrolide antibiotic, an effective dose of antacid and a pharmaceutical acceptable carrier, wherein the pharmaceutical composition is present in the forms of suspension, powder, or effervescent tablet.

The pharmaceutical composition in the forms of suspension, powder, or effervescent tablet can be dissolved rapidly in water to form a liquid formula.

The present invention further provides a method for preparing a pharmaceutical composition for the eradication of *Helicobacter pylori,* which comprises of the following steps: i) mixing an effective dose of β-lactam antibiotic and a pharmaceutical acceptable carrier to form a first mixture; ii) mixing an effective dose of macrolide antibiotic and a pharmaceutical acceptable carrier to form a second mixture; iii) mixing the first mixture and the second mixture with an antacid to form a third mixture; and iv) mixing the third mixture with a glidant, an anti-bacterial agent, a lubricants, and a suspending agent to form the pharmaceutical composition in powder form or suspension form.

The present invention provides another method for preparing a pharmaceutical composition for the eradication of *Helicobacter pylori,* which comprises of the following steps: i) mixing a antacid, a suspending agent, an effective dose of macrolide antibiotic and a pharmaceutical acceptable carrier to form a first mixture; ii) mixing an effective dose of β-lactam antibiotic and a pharmaceutical acceptable carrier including a lubricant to form a second mixture; and iii) mixing the first mixture and the second mixture evenly to form the pharmaceutical composition in powder form or suspension form.

The present invention further provides another method for preparing a pharmaceutical composition for the eradication of *Helicobacter pylori,* which comprises of the following steps: i) mixing an effective dose of β-lactam antibiotic, an effective dose of macrolide antibiotic, and a pharmaceutical acceptable carrier to form a first mixture; ii) mixing an effective dose of an antacid, an anti-bacterial agent and a pharmaceutical acceptable carrier to form a second mixture; iii) mixing the first mixture and the second mixture evenly with a foaming agent to formulate; and iv) incorporating a glidant for compression formulation to produce the pharmaceutical composition in effervescent tablet form. Among the above methods, the pharmaceutical composition in the forms of suspension, powder, or effervescent tablet can be dissolved rapidly in water as a liquid formula.

The antacid described in the abovementioned pharmaceutical compositions and the methods can be a H₂ Blocker or a proton pump inhibitor (PPI). PPI is omeprazole, lansoprazole, esomeprazole, pantoprazole, tenatoprazole rabeprazole, or an enantiomer, isomer, free base, salt, or mixture thereof; and H₂ blocker is cimetidine, ranitidine, famotidine, nizatidine, or an enantiomer, isomer, free base, salt, or mixture thereof. In addition, an effective dose of alkaline substance can be added to increase the pH of the stomach when the PPI antacid is used, wherein the alkaline substance is carbonate or bicarbonate such as sodium bicarbonate or magnesium carbonate.

The β-lactam antibiotic is penicillin, phenoxypenicillin, oxacillin, cloxacillin, dicloxacillin, flucloxacillin, nafcillin, ampicillin, bacampicillin, amoxicillin, carbenicillin, ticarcillin, furbucillin, piperacillin, cefalotin, cefaloridin, cefalexin, cefazolin, cefradine, cefadroxil, cefamandole, cefuroxime, cefoxitin, cefotaxime, cefoperazone, ceftriaxone, ceftazidime, latamoxef, cefepime, cefpirome, cefclidin, aztreouam, imipenem, or meropenem. The macrolide antibiotic is erythromycin, spiramycin, kitasamycin, midecamycin, jasamycin, roxithromycin, clarithromycin, azithromycin, tetracycline, oxytetracycline, demeclocycline, chlortetracycline, doxycycline, lymecycline, meclocycline, methacycline, minocycline, or rolitetracycline. The pharmaceutical composition further comprises one or more pharmaceutically acceptable carriers selected from the group consisting of excipients, flavor enhancers, effervescent agents, glidants, anti-bacterial agents, suspending agents, disintegrants, lubricants, fillers, binders, diluents, absorption enhancers and mixtures thereof.

The pharmaceutical composition in the suspension form and its preparing method include ranitidine/ bismuth citrate, clarithromycin, and amoxicillin. The preferred dosages are 290 mg for ranitidine/ bismuth citrate, 500 mg for clarithromycin, and 1 g for amoxicillin. The preferred composition is amoxicillin, clarithromycin, and famotidine, wherein the preferred dosages are 1g for amoxicillin, 500 mg for clarithromycin, and 20 mg for famotidine.

In addition, the pharmaceutically acceptable carriers are selected from the group consisting of flavor enhancer, glidant, anti-bacterial agent, suspending agent, disintegrant, and lubricants. Among them, flavor enhancers include mannitol, fructose, xylitol, dextrose, lactose, sucrose and glucose; suspending agents are selected from the group consisting of xanthan gum, acacia, agar, carbomer, carboxymethylcellulose calcium, carboxymethylcellulose Sodium, carrageenan, microcrystalline cellulose, ceratonia, gelatin, hydroxyethyl cellulose, hydroxyethylmethyl cellulose, hydroxypropyl cellulose, hypromellose, methylcellulose and hydroxypropylmethyl cellulose; glidants are selected from the group consisting of colloidal silicon dioxide, and talc; lubricants are selected from the group consisting of magnesium stearate and talc; and disintegrants are selected from the group consisting of povidone and crospovidone.

The pharmaceutical composition in the form of effervescent tablet and powder and its preparing method include amoxicillin, clarithromycin, and omeprazole. The preferred dosages are 1 g for amoxicillin, 500 mg for clarithromycin, and 20 mg for omeprazole; and the alkaline substance is carbonate or bicarbonate. In addition, the pharmaceutically acceptable carriers are selected from the group consisting of excipients, effervescent agents, flavor enhancers, glidants, and anti-bacterial agents. Among them, flavor enhancer is selected from the group consisting of mannitol, fructose, xylitol, dextrose, lactose, sucrose and glucose; excipient is selected from the group consisting of microfine cellulose, microcrystalline cellulose, carboxymethylcellulose calcium, carboxymethylcellulose sodium, hydroxypropyl cellulose, polyvinyl pyrrolidone and sodium starch glycollate. The effervescent agent is composed of alkaline substances and organic acids, wherein the alkaline substance is carbonate or bicarbonate; the organic acid is monoalkali metal citrate such as anhydrous citric acid, and malic acid. The glidant is selected from the group consisting of colloidal silicon dioxide, and talc.

On the other hand, the present invention has alkaline substance such as carbonate or bicarbonate supplemented into the pharmaceutical composition when PPI is used as antacid, which can increase the pH in the stomach, to protect the degradation of acid-labile antibiotics or PPI to further increase the bioavailability of the medication for the purpose of *Helicobacter pylori* eradication.

Furthermore, the method for preparing a pharmaceutical composition in the suspension form according to the present invention has mixed the β-lactam antibiotic and the macrolide antibiotic with a pharmaceutically acceptable carrier respectively to form a first mixture and a second mixture, followed by mixing with antacids. The purpose is to provide segregation among the β-lactam antibiotic, the macrolide antibiotic and PPI, thus these three compounds would not directly contact to each other, in order to improve the stability of each compounds. Similarly, the method for preparing a pharmaceutical composition in the powder form according to the invention also mixed the antacid, suspending agent and a macrolide antibiotic with a pharmaceutically acceptable carrier first, then mixed with another mixture of β-lactam antibiotic and a pharmaceutically acceptable carrier including lubricant to prevent the instability caused by directly contact of the antibiotics and antacids. In addition, the method for preparing a pharmaceutical composition in the effervescent tablet according to the present invention also produced a first mixture of β-lactam antibiotic and a pharmaceutically acceptable carrier, which can be mixed with antacids to prevent the instability.

According to the present invention, the pharmaceutical composition for the eradication of *Helicobacter pylori* in the forms of effervescent tablet, suspension or powder has formulated all the medications from triple therapy into one form for easy carriage. In addition, the pharmaceutical composition in the forms of effervescent tablet, suspension or powder is dissolved in a liquid for ingestion, which makes patients easy to swallow, to further increase the compliance. In this way, the pharmaceutical composition can be absorbed rapidly, and has advantages such as good stability, fast disintegrating, rapid absorption, easy to swallow liquid form, tolerant to gastrointestinal tract, easy to carry, flavor changeable (enhance flavor, easy for swallowing), applicable in large scale of medication, better absorption stability and more predictable on the outcome of therapy. At the same time, the preparation method has made the pharmaceutical composition to have a better stability and the bioavailability increased.

The present invention is further explained in the following embodiment illustration and examples. Those examples below should not, however, be considered to limit the scope of the invention, it is contemplated that modifications will readily occur to those skilled in the art, which modifications will be within the spirit of the invention and the scope of the appended claims.

### BRIEF DESCRIPTION OF THE DRAWINGS

Fig. 1 is the famotidine stability analysis of the pharmaceutical composition in suspension form for the eradication of *Helicobacter pylori* according to the present invention.

Fig. 2 is the amoxicillin stability analysis of the pharmaceutical composition in suspension form for the eradication of *Helicobacter pylori* according to the present invention.

Fig. 3 is the clarithromycin stability analysis of the pharmaceutical composition in suspension form for the eradication of *Helicobacter pylori* according to the present invention.

Fig. 4 is the blood concentration of amoxicillin in effervescent tablet form of the pharmaceutical composition after ingested by animals in the embodiment.

Fig. 5 is the blood concentration of claritharmycin in effervescent tablet form of the pharmaceutical composition after ingested by animals in the embodiment.

Fig. 6 is the blood concentration of omeprazole in effervescent tablet form of the pharmaceutical composition after ingested by animals in the embodiment.

### DETAILED DESCRIPTION OF THE PREFERRED EMBODIMENT

The present invention formulates multiple medications of the traditional triple therapy into one pharmaceutical composition for the eradication of *Helicobacter pylori* in the forms of effervescent tablet, suspension, and powders, and dissolves the pharmaceutical composition in a liquid form for ingestion to improve the patient compliance. By supplementing alkaline substances such as carbonate or bicarbonate, the pH of stomach can be adjusted to a more alkaline condition to prevent the instability of antibiotics or the degradation of PPI under acidic conditions. The drugs can function well in the stomach and their bio-availability can be increased. The *Helicobacter pylori* can be eradicated after ingestion of the pharmaceutical composition for several days.

The pharmaceutical composition comprising an effective dose of β-lactam antibiotic, an effective dose of macrolide antibiotic, an effective dose of antacid and a pharmaceutical acceptable carrier, wherein the pharmaceutical composition is in the forms of suspension, powder or effervescent tablet.

Among them, the antacid can be a H₂ Blocker or a proton pump inhibitor (PPI). PPI includes, but is not limited to, omeprazole, lansoprazole, esomeprazole, pantoprazole, tenatoprazole or rabeprazole; and H₂ Blocker includes, but is not limited to, cimetidine, ranitidine, famotidine, or nizatidine. The definition of "PPI" or "H₂ Blocker" also means that the antacids of the present invention may be administered, if desired, in the form of salts, free base, enantiomers, isomers, derivatives and the like, provided the salt, free base, enantiomer, isomer,or derivative is suitable pharmacologically, that is, effective in the present methods, combinations and compositions. Salts, free base, enantiomers, isomers and other derivatives of the antacids may be prepared using standard procedures known to those skilled in the art of synthetic organic chemistry and described, for example, by J. March, Advanced Organic Chemistry; Reactions, Mechanisms and Structure, 4th Ed. (New York: Wiley-Interscience, 1992). In addition, an effective dose of alkaline substance can be added to increase the pH of the stomach when the antacid is a PPI.

The β-lactam antibiotic is penicillins, phenoxypenicillins, oxacillin, cloxacillin, dicloxacillin, flucloxacillin, nafcillin, ampicillin, bacampicillin, amoxicillin, carbenicillin, ticarcillin, furbucillin, piperacillin, cefalotin, cefaloridin, cefalexin, cefazolin, cefradine, cefadroxil, cefamandole, cefuroxime, cefoxitin, cefotaxime, cefoperazone, ceftriaxone, ceftazidime, latamoxef, cefepime, cefpirome, cefclidin, aztreouam, imipenem, or meropenem. The macrolide antibiotic is erythromycin, spiramycin, kitasamycin, midecamycin, jasamycin, roxithromycin, clarithromycin, azithromycin, tetracycline, oxytetracycline, demeclocycline, chlortetracycline, doxycycline, lymecycline, meclocycline, methacycline, minocycline or rolitetracycline. The alkaline substance is carbonate or bicarbonate such as sodium bicarbonate or magnesium carbonate. The one or more pharmaceutically acceptable carriers are selected from the group consisting of excipients, effervescent agents, flavor enhancers, glidants, anti-bacterial agents, suspending agents, disintegrants, lubricants, fillers, binders, diluents, absorption enhancers and mixtures thereof, and are not limited to. The embodiments for preparing the pharmaceutical composition for the eradication of *Helicobacter pylori* in the forms of effervescent tablet, suspension and powder are listed below respectively.

### Example 1 Production of the pharmaceutical composition for the eradication of Helicobacter pylori in the suspension form

The active ingredients of the pharmaceutical composition for the eradication of *Helicobacter pylori* in the suspension form include 20 mg of famotidine, 500 mg of clarithromycin and 1 g of amoxicillin, where famotidine can be replaced with 290 mg of ranitidine/ bismuth citrate, wherein famotidine and ranitidine are H₂ Blocker. Famotidine was used in the embodiment of the present invention, which comprised of 20 mg of famotidine, 500 mg of clarithromycin and 1 g of amoxicillin, Ac-Di-Sol as disintegrant, colloidal silicon dioxide as glidant, xanthan gum, carboxymethylcellulose sodium and hydroxypropyl methylcellulose as suspending agents, sodium benzoate as anti-bacterial agent, magnesium stearate as lubricant. The composition and preparation method is described in the following table.

(1) Amoxicillin was prepared by passing through a sieve (number 60), followed by addition of Ac-Di-Sol and passing through the sieve again;

(2) Clarithromycin was prepared by passing through a sieve (number 60),

| Ingredients of pharmaceutical composition in the suspension form | Theoretical Weight (mg)/20ml | % | Formulations |
|---|---|---|---|
| Amoxycillin trihydrate | 1000.0 | 48.9% | 250∼4000mg |
| Clarithromycin | 500.0 | 24.5% | 125∼2000mg |
| Famotidine | 20.0 | 1.0% | 5∼80mg |
| Ac-Di-Sol | 30.5 | 1.5 % | 0.5∼5% |
| Xanthan gum | 10.1 | 0.5% | 0.2∼15% |
| Carboxymethylcellulose sodium salt | 20.0 | 1.0% | 2∼15% |
| Hydroxypropyl methylcellulose (HPMC) | 83.0 | 4.1% | 2∼15% |
| Sodium benzoate | 20.3 | 1.0% | 0.02∼1.5% |
| Silica gel | 216.7 | 10.6% | 5∼5% |
| Hydrophobic colloidal silica | 6.0 | 0.3% | 0.1∼2% |
| Magnesium stearate | 4.0 | 0.2% | 0.25∼5% |
| Aspartame | 75.0 | 3.7% | 2∼15% |
| grape flavour | 58.6 | 2.9% | 2∼15% |
| Total weight | 2044.2 | 100.0% | |

followed by addition of carboxymethylcellulose sodium and hydroxypropyl methylcellulose and passing through the sieve again;

(3) Clarithromycin from step (2) and amoxicillin from step (1) were mixed evenly;

(4) Famotidine was added into the above mixture and passed through a sieve (number 60) to form famotidine/ amoxicillin/ clarithromycin granules;

(5) Hydrophobic colloidal silica, xanthan gum, sodium benzoate and magnesium stearate were added into famotidine/amoxicillin/clarithromycin granules respectively, and passed through a sieve (number 60) to form famotidine/ amoxicillin/ clarithromycin suspension.

The supplements for the suspension type pharmaceutical composition are not limited to the abovementioned substances, which may also include suspending agent, glidant, lubricants and disintegrants. Suspending agents include, but not limited to, acacia, agar, carbomer, carboxymethylcellulose calcium, carboxymethylcellulose sodium, carrageenan, microcrystalline cellulose, ceratonia, gelatin, hydroxyethyl cellulose, hydroxyethylmethyl cellulose, hydroxypropyl cellulose, hypromellose, methylcellulose or hydroxypropylmethyl cellulose; glidants include, but not limited to, colloidal silicon dioxide, or talc; lubricants include, but not limited to, magnesium stearate or talc; and disintegrants include, but not limited to, povidone or crospovidone. Other combination of the ingredients and the preparation methods are also listed below.

1. The composition and preparation method for suspension pharmaceutical compositions containing antibiotics of clarithromycin and amoxicillin, and PPI of omeprazole. The composition and preparation method is described in the following table.

| Ingredients of pharmaceutical composition in the suspension form | Theoretical Weight (mg)/20ml | % |
|---|---|---|
| Amoxycillin trihydrate | 1000.0 | 48.1% |
| Clarithromycin | 500.0 | 24.1% |
| Omeprazole | 20.0 | 1.0% |
| Ac-Di-Sol | 30.5 | 1.5% |
| Xanthan gum | 10.1 | 0.5 % |
| Carboxymethylcellulose sodium salt | 20.0 | 1.0% |
| Hydroxypropyl methylcellulose(HPMC) | 115.0 | 5.5 % |
| Sodium benzoate | 20.3 | 1.0% |
| Silica gel | 216.7 | 10.4% |
| Hydrophobic colloidal silica | 6.0 | 0.3% |
| Magnesium stearate | 4.0 | 0.2% |
| Aspartame | 75.0 | 3.6% |
| lemon flavour | 60.0 | 2.9% |
| Total weight | 2077.6 | 100.0% |

(1) Amoxicillin was prepared by passing through a sieve (number 60), followed by addition of Ac-Di-Sol and passing through the sieve again;

(2) Clarithromycin was prepared by passing through a sieve (number 60), followed by addition of carboxymethylcellulose sodium and hydroxypropyl methylcellulose and passing through the sieve again;

(3) Clarithromycin from step (2) and amoxicillin from step (1) were mixed evenly;

(4) Omeprazole was added into the above mixture and passed through a sieve (number 60) to form omeprazole / amoxicillin/ clarithromycin granules;

(5) Hydrophobic colloidal silica, xanthan gum, sodium benzoate and magnesium stearate were added into omeprazole/amoxicillin/clarithromycin granules respectively, and passed through a sieve (number 60) to form omeprazole/ amoxicillin/ clarithromycin suspension.

2. The composition and preparation method for suspension pharmaceutical compositions containing antibiotics of clarithromycin and amoxicillin, and PPI of ranitidine/ bismuth citrate. The composition and preparation method is described in the following table.

| Ingredients of pharmaceutical composition in the suspension form | Theoretical Weight (mg)/20ml | % |
|---|---|---|
| Amoxycillin trihydrate | 1000.0 | 42.1% |
| Clarithromycin | 500.0 | 21.0% |
| Ranitidine | 162.0 | 6.8% |
| bismuth citrate | 128.0 | 5.4% |
| Ac-Di-Sol | 30.5 | 1.3% |
| Xanthan gum | 10.1 | 0.4% |
| Carboxymethylcellulose sodium salt | 20.0 | 0.8% |
| Hydroxypropyl methylcellulose(HPMC) | 115.0 | 4.8% |
| Sodium benzoate | 20.3 | 0.9% |
| Silica gel | 216.7 | 9.1% |
| Hydrophobic colloidal silica | 6.0 | 0.3% |
| Magnesium stearate | 4.0 | 0.2% |
| Aspartame | 75.0 | 3.2% |
| lemon flavour | 90.0 | 3.8% |
| Total weight | 2377.6 | 100.0% |

(1) Amoxicillin was prepared by passing through a sieve (number 60), followed by addition of Ac-Di-Sol and passing through the sieve again;

(2) Clarithromycin was prepared by passing through a sieve (number 60), followed by addition of carboxymethylcellulose sodium and hydroxypropyl methylcellulose and passing through the sieve again;

(3) Clarithromycin from step (2) and amoxicillin from step (1) were mixed evenly;

(4) Ranitidine/ bismuth citrate were added into the above mixture and passed through a sieve (number 60) to form ranitidine/ bismuth citrate / amoxicillin/ clarithromycin granules;

(5) Hydrophobic colloidal silica, xanthan gum, sodium benzoate and magnesium stearate were added into ranitidine/ bismuth citrate/ amoxicillin/ clarithromycin granules respectively, and passed through a sieve (number 60) to form ranitidine/ bismuth citrate/ amoxicillin/ clarithromycin suspension.

3. The composition and preparation method for suspension pharmaceutical compositions containing antibiotics of clarithromycin and amoxycillin, and PPI of cimetidine. The composition and preparation method is described in the following table.

| Ingredients of pharmaceutical composition in the suspension form | Theoretical Weight (mg)/20ml | % |
|---|---|---|
| Amoxycillin trihydrate | 1000.0 | 44.5% |
| Clarithromycin | 500.0 | 22.2% |
| Cimetidine | 162.0 | 7.2% |
| Ac-Di-Sol | 30.5 | 1.4% |
| Xanthan gum | 10.1 | 0.4% |
| Carboxymethylcellulose sodium salt | 20.0 | 0.9% |
| Hydroxypropylmethylcellulose(HPMC) | 115.0 | 5.1% |
| Sodium benzoate | 20.3 | 0.9% |
| Silica gel | 216.7 | 9.6% |
| Hydrophobic colloidal silica | 6.0 | 0.3% |
| Magnesium stearate | 4.0 | 0.2% |
| Aspartame | 75.0 | 3.3% |
| lemon flavour | 90.0 | 4.0% |
| Total weight | 2249.6 | 100.0% |

(1) Amoxicillin was prepared by passing through a sieve (number 60), followed by addition of Ac-Di-Sol and passing through the sieve again;

(2) Clarithromycin was prepared by passing through a sieve (number 60), followed by addition of carboxymethylcellulose sodium and hydroxypropyl methylcellulose and passing through the sieve again;

(3) Clarithromycin from step (2) and amoxicillin from step (1) were mixed evenly;

(4) Cimetidine was added into the above mixture and passed through a sieve (number 60) to form cimetidine / amoxicillin/ clarithromycin granules;

(5) Hydrophobic colloidal silica, xanthan gum, sodium benzoate and magnesium stearate were added into cimetidine/ amoxicillin/ clarithromycin granules respectively, and passed through a sieve (number 60) to form cimetidine/ amoxicillin/ clarithromycin suspension.

4. The composition and preparation method for suspension pharmaceutical compositions containing antibiotics of amoxicillin and tetracycline, and PPI of omeprazole. The composition and preparation method is described in the following table.

| Ingredients of pharmaceutical composition in the suspension form | Theoretical Weight (mg)/20ml | % |
|---|---|---|
| Amoxycillin trihydrate | 1000.0 | 47.4% |
| Tetracycline | 500.0 | 23.7% |
| Omeprazole | 20.0 | 0.9% |
| Ac-Di-Sol | 30.5 | 1.4% |
| Xanthan gum | 10.1 | 0.5% |
| Carboxymethylcellulose sodium salt | 20.0 | 0.9% |
| Hydroxypropyl methylcellulose(HPMC) | 115.0 | 5.5% |
| Sodium benzoate | 20.3 | 1.0% |
| Silica gel | 216.7 | 10.3% |
| Hydrophobic colloidal silica | 6.0 | 0.3% |
| Magnesium stearate | 4.0 | 0.2% |
| Aspartame | 75.0 | 3.6% |
| lemon flavour | 90.0 | 4.3% |
| Total weight | 2107.6 | 100.0% |

(1) Amoxicillin was prepared by passing through a sieve (number 60), followed by addition of Ac-Di-Sol and passing through the sieve again;

(2) Tetracycline was prepared by passing through a sieve (number 60), followed by addition of carboxymethylcellulose sodium and hydroxypropyl methylcellulose and passing through the sieve again;

(3) Tetracycline from step (2) and amoxicillin from step (1) were mixed evenly;

(4) Omeprazole was added into the above mixture and passed through a sieve (number 60) to form omeprazole / amoxicillin/ tetracycline granules;

(5) Hydrophobic colloidal silica, xanthan gum, sodium benzoate and magnesium stearate were added into omeprazole/amoxicillin/tetracycline granules respectively, and passed through a sieve (number 60) to form omeprazole/ amoxicillin/ tetracycline suspension.

5. The composition and preparation method for suspension pharmaceutical compositions containing antibiotics of clarithromycin and tetracycline, and PPI of omeprazole. The composition and preparation method is described in the following table.

| Ingredients of pharmaceutical composition in the suspension form | Theoretical Weight (mg)/20ml | % |
|---|---|---|
| Clarithromycin | 500 | 31.1% |
| Tetracycline | 500 | 31.1% |
| Omeprazole | 20 | 1.2% |
| Ac-Di-Sol | 30.5 | 1.9% |
| Xanthan gum | 10.1 | 0.6% |
| Carboxymethylcellulose sodium salt | 20 | 1.2% |
| Hydroxypropyl methylcellulose(HPMC) | 115 | 7.2% |
| Sodium benzoate | 20.3 | 1.3% |
| Silica gel | 216.7 | 13.5% |
| Hydrophobic colloidal silica | 6 | 0.4% |
| Magnesium stearate | 4 | 0.2% |
| Aspartame | 75 | 4.7% |
| lemon flavour | 90 | 5.6% |
| Total weight | 1607.6 | 100.0% |

(1) Tetracycline was prepared by passing through a sieve (number 60), followed by addition of Ac-Di-Sol and passing through the sieve again;

(2) Clarithromycin was prepared by passing through a sieve (number 60), followed by addition of carboxymethylcellulose sodium and hydroxypropyl methylcellulose and passing through the sieve again;

(3) Clarithromycin from step (2) and tetracycline from step (1) were mixed evenly;

(4) Omeprazole was added into the above mixture and passed through a sieve (number 60) to form omeprazole / tetracycline/ clarithromycin granules;

(5) Hydrophobic colloidal silica, xanthan gum, sodium benzoate and magnesium stearate were added into omeprazole/ tetracycline/ clarithromycin granules respectively, and passed through a sieve (number 60) to form omeprazole/ tetracycline/ clarithromycin suspension.

6. The composition and preparation method for suspension pharmaceutical compositions containing antibiotics of amoxicillin and tetracycline, and PPI of famotidine. The composition and preparation method is described in the following table.

| Ingredients of pharmaceutical composition in the suspension form | Theoretical Weight (mg)/20ml | % |
|---|---|---|
| Amoxycillin trihydrate | 1000.0 | 47.4% |
| Tetracycline | 500.0 | 23.7% |
| Famotidine | 20.0 | 0.9% |
| Ac-Di-Sol | 30.5 | 1.4% |
| Xanthan gum | 10.1 | 0.5% |
| Carboxymethylcellulose sodium salt | 20.0 | 0.9% |
| Hydroxypropyl methylcellulose(HPMC) | 115.0 | 5.5% |
| Sodium benzoate | 20.3 | 1.0% |
| Silica gel | 216.7 | 10.3% |
| Hydrophobic colloidal silica | 6.0 | 0.3% |
| Magnesium stearate | 4.0 | 0.2% |
| Aspartame | 75.0 | 3.6% |
| lemon flavour | 90.0 | 4.3% |
| Total weight | 2107.6 | 100.0% |

(1) Amoxicillin was prepared by passing through a sieve (number 60), followed by addition of Ac-Di-Sol and passing through the sieve again;

(2) Tetracycline was prepared by passing through a sieve (number 60), followed by addition of carboxymethylcellulose sodium and hydroxypropyl methylcellulose and passing through the sieve again;

(3) Tetracycline from step (2) and amoxicillin from step (1) were mixed evenly;

(4) Famotidine was added into the above mixture and passed through a sieve (number 60) to form famotidine / amoxicillin/ tetracycline granules;

(5) Hydrophobic colloidal silica, xanthan gum, sodium benzoate and magnesium stearate were added into famotidine/ amoxicillin/ tetracycline granules respectively, and passed through a sieve (number 60) to form famotidine/ amoxicillin/ tetracycline suspension.

7. The composition and preparation method for suspension pharmaceutical compositions containing antibiotics of clarithromycin and tetracycline, and PPI of famotidine. The composition and preparation method is described in the following table.

| Ingredients of pharmaceutical composition in the suspension form | Theoretical Weight (mg)/20ml | % |
|---|---|---|
| Clarithromycin | 500.0 | 31.1% |
| Tetracycline | 500.0 | 31.1% |
| Famotidine | 20.0 | 1.2% |
| Ac-Di-Sol | 30.5 | 1.9% |
| Xanthan gum | 10.1 | 0.6% |
| Carboxymethylcellulose sodium salt | 20.0 | 1.2% |
| Hydroxypropyl methylcellulose(HPMC) | 115.0 | 7.2% |
| Sodium benzoate | 20.3 | 1.3% |
| Silica gel | 216.7 | 13.5% |
| Hydrophobic colloidal silica | 6.0 | 0.4% |
| Magnesium stearate | 4.0 | 0.2% |
| Aspartame | 75.0 | 4.7% |
| lemon flavour | 90.0 | 5.6% |
| Total weight | 1607.6 | 100.0% |

(1) Tetracycline was prepared by passing through a sieve (number 60), followed by addition of Ac-Di-Sol and passing through the sieve again;

(2) Clarithromycin was prepared by passing through a sieve (number 60), followed by addition of carboxymethylcellulose sodium and hydroxypropyl methylcellulose and passing through the sieve again;

(3) Clarithromycin from step (2) and tetracycline from step (1) were mixed evenly;

(4) Famotidine was added into the above mixture and passed through a sieve (number 60) to form famotidine / clarithromycin / tetracycline granules;

(5) Hydrophobic colloidal silica, xanthan gum, sodium benzoate and magnesium stearate were added into famotidine/ clarithromycin / tetracycline granules respectively, and passed through a sieve (number 60) to form famotidine/ clarithromycin / tetracycline suspension.

8. The composition and preparation method for suspension pharmaceutical compositions containing antibiotics of amoxicillin and tetracycline, and PPI of ranitidine/ bismuth citrate. The composition and preparation method is described in the following table.

| Ingredients of pharmaceutical composition in the suspension form | Theoretical Weight (mg)/20ml | % |
|---|---|---|
| Amoxycillin trihydrate | 1000.0 | 42.1% |
| Tetracycline | 500.0 | 21.0% |
| Ranitidine | 162.0 | 6.8% |
| bismuth citrate | 128.0 | 5.4% |
| Ac-Di-Sol | 30.5 | 1.3% |
| Xanthan gum | 10.1 | 0.4% |
| Carboxymethylcellulose sodium salt | 20.0 | 0.8% |
| Hydroxypropyl methylcellulose(HPMC) | 115.0 | 4.8% |
| Sodium benzoate | 20.3 | 0.9% |
| Silica gel | 216.7 | 9.1% |
| Hydrophobic colloidal silica | 6.0 | 0.3% |
| Magnesium stearate | 4.0 | 0.2% |
| Aspartame | 75.0 | 3.2% |
| lemon flavour | 90.0 | 3.8% |
| Total weight | 2377.6 | 100.0% |

(1) Amoxicillin was prepared by passing through a sieve (number 60), followed by addition of Ac-Di-Sol and passing through the sieve again;

(2) Tetracycline was prepared by passing through a sieve (number 60), followed by addition of carboxymethylcellulose sodium and hydroxypropyl methylcellulose and passing through the sieve again;

(3) Tetracycline from step (2) and amoxicillin from step (1) were mixed evenly;

(4) Ranitidine/ bismuth citrate were added into the above mixture and passed through a sieve (number 60) to form ranitidine/ bismuth citrate / amoxicillin/ tetracycline granules;

(5) Hydrophobic colloidal silica, xanthan gum, sodium benzoate and magnesium stearate were added into ranitidine/ bismuth citrate/ amoxicillin/ tetracycline granules respectively, and passed through a sieve (number 60) to form ranitidine/ bismuth citrate/ amoxicillin/ tetracycline suspension.

9. The composition and preparation method for suspension pharmaceutical compositions containing antibiotics of clarithromycin and tetracycline, and PPI of ranitidine/ bismuth citrate. The composition and preparation method is described in the following table.

| Ingredients of pharmaceutical composition in the suspension form | Theoretical Weight (mg)/20ml Theoretical Weight | % |
|---|---|---|
| Clarithromycin | 500.0 | 26.6% |
| Tetracycline | 500.0 | 26.6% |
| Ranitidine | 162.0 | 8.6% |
| bismuth citrate | 128.0 | 6.8% |
| Ac-Di-Sol | 30.5 | 1.6% |
| Xanthan gum | 10.1 | 0.5% |
| Carboxymethylcellulose sodium salt | 20.0 | 1.1 % |
| Hydroxypropyl methylcellulose(HPMC) | 115.0 | 6.1% |
| Sodium benzoate | 20.3 | 1.1 % |
| Silica gel | 216.7 | 11.5% |
| Hydrophobic colloidal silica | 6.0 | 0.3% |
| Magnesium stearate | 4.0 | 0.2% |
| Aspartame | 75.0 | 4.0% |
| lemon flavour | 90.0 | 4.8% |
| Total weight | 1877.6 | 100.0% |

(1) Tetracycline was prepared by passing through a sieve (number 60), followed by addition of Ac-Di-Sol and passing through the sieve again;

(2) Clarithromycin was prepared by passing through a sieve (number 60), followed by addition of carboxymethylcellulose sodium and hydroxypropyl methylcellulose and passing through the sieve again;

(3) Clarithromycin from step (2) and tetracycline from step (1) were mixed evenly;

(4) Ranitidine/ bismuth citrate were added into the above mixture and passed through a sieve (number 60) to form ranitidine/ bismuth citrate / clarithromycin / tetracycline granules;

(5) Hydrophobic colloidal silica, xanthan gum, sodium benzoate and magnesium stearate were added into ranitidine/ bismuth citrate/ clarithromycin / tetracycline granules respectively, and passed through a sieve (number 60) to form ranitidine/ bismuth citrate/ clarithromycin / tetracycline suspension.

### Example 2 Production of the pharmaceutical composition for the eradication of Helicobacter pylori in the effervescent tablet form

The active ingredients of the pharmaceutical composition for the eradication of *Helicobacter pylori* in the effervescent tablet form include 20 mg of omeprazole, 500 mg of clarithromycin and 1 g of amoxicillin, effervescent agents such as sodium bicarbonate and anhydrous citric acid, anti-bacterial agent such as sodium benzoate, glidant such as colloidal silicon dioxide, suspending agents such as carboxymethylcellulose sodium and hydroxypropyl methylcellulose, and flavor enhancer. The composition and preparation method is described in the following table.

| Ingredients of pharmaceutical composition in the effervescent tablet form | Theoretical Weight (mg)/20ml | % | Formulations |
|---|---|---|---|
| Amoxycillin trihydrate | 101.6 | 7.6% | 250∼1000mg |
| Clarithromycin | 51.2 | 3.8% | 125∼500mg |
| Omeprazole | 4.0 | 0.3% | 5∼20mg |
| Mannitol | 700 | 52.2% | 15∼75% |
| Hydroxypropyl methylcellulose(HPMC) | 42 | 3.1% | 2∼15% |
| Carboxymethylcellulose sodium salt(CMC) | 14.5 | 1.1 % | 2∼15% |
| Xanthan gum | 3 | 0.2% | 0.2∼15% |
| Sodium benzoate | 14 | 1.0% | 0.02∼1.5% |
| Aspartame | 75 | 5.6% | 1∼25% |
| Sodium bicarbonate | 168 | 12.5% | 5∼25% |
| Citric acid | 84 | 6.3% | |
| Polyvinylpyrrolidone | 2 | 0.1 % | 0.1∼8% |
| Magnesium Stearate | 3.5 | 0.3% | 0.2∼5% |
| Lemon flavour powder | 70 | 5.2% | 2∼15% |
| Hydrophobic colloidal silica | 7 | 0.5 % | 0.1∼0.7% |
| total | 1339.8 | 100.0% | |

(1) 500 mg of clarithromycin and 1 g of amoxicillin were prepared by passing through a sieve (number 60), followed by addition of mannitol and passing through the sieve again;

(2) 20 mg of omeprazole and hydroxypropyl methylcellulos were mixed and passed through the sieve (number 60);

(3) Sodium bicarbonate and anhydrous citric acid methylcellulos were mixed and passed through the sieve (number 40);

(4) Granules from step (1), step (2) and step (3) were mixed evenly and passed through the sieve (number 40);

(5) Polyvinyl pyrrolidone (PVP) was resuspended and mixed evenly into 25 ml of 95% ethanol;

(6) PVP solution was added into mixed granulates from step (4) for granulation;

(7) Granules from step (6) were dried at 40°C, followed by addition of magnesium stearate and hydrophobic colloidal silica, and passed through a sieve (number 20) to compress and form amoxicillin/ clarithromycin/ omeprazole effervescent tablets.

The supplements for the effervescent tablet type pharmaceutical composition are not limited to the abovementioned substances, which may also include flavor enhancers, effervescent agents, and excipients, wherein flavor enhancers include, but not limited to, mannitol, fructose, xylitol, dextrose, lactose, sucrose, and glucose; effervescent agents can be organic acids such as carbonate or bicarbonate salts such as sodium bicarbonate, magnesium carbonate, and monoalkali metal citrate such as anhydrous citric acid, and malic acid. Excipients include, but not limited to, microfine cellulose, hydroxypropyl cellulose, polyvinyl pyrrolidone, and sodium starch glycollate.

### Example 3 Production of the pharmaceutical compositions for the eradication of Helicobacter pylori in the powder form

| Ingredients of pharmaceutical composition in the powder form | Theoretical Weight (mg)/20ml | % | Range of Ingredients |
|---|---|---|---|
| Amoxycillin trihydrate | 101.6 | 11.5% | 250∼1000mg |
| Clarithromycin | 51.2 | 5.8% | 125∼500mg |
| Omeprazole | 4.0 | 0.5% | 5∼20mg |
| Mannitol | 350.0 | 39.6% | 15∼75% |
| Hydroxypropyl methylcellulose(HPMC) | 42.0 | 4.7% | 2∼15% |
| Xanthan gum | 3.0 | 0.3% | 0.2∼15% |
| Sodium benzoate | 14.0 | 1.6% | 0.02∼1.5% |
| Aspartame | 75.0 | 8.5 % | 1∼25% |
| Sodium bicarbonate | 168.0 | 19.0% | 5∼25% |
| Polyvinyl pyrrolidone | 2.0 | 0.2% | 0.1∼8% |
| Magnesium Stearate | 3.5 | 0.4% | 0.2∼5% |
| Lemon flavour powder | 70.0 | 7.9% | 2∼15% |
| total | 884.3 | 100.0% | |

(1) Omeprazole was prepared by passing through a sieve (number 60), followed by addition of hydroxypropyl methylcellulose and passing through the sieve again;

(2) Clarithromycin was prepared by passing through a sieve (number 60), followed by addition of mannitol and passing through the sieve again;

(3) Clarithromycin from step (2) and omeprazole mixture from step (1) were mixed evenly;

(4) Amoxicillin was added into the above mixture, mixed evenly and passed through a sieve (number 60) to form omeprazole / amoxicillin/ clarithromycin granules;

(5) Xanthan gum, sodium benzoate, flavor enhancer, PVP and magnesium stearate were added into omeprazole/ amoxicillin/ clarithromycin granules respectively, and passed through a sieve (number 60) to form omeprazole/ amoxicillin/ clarithromycin powder.

### Example 4 Stability analysis of the pharmaceutical composition for the eradication of Helicobacter pylori in the suspension form

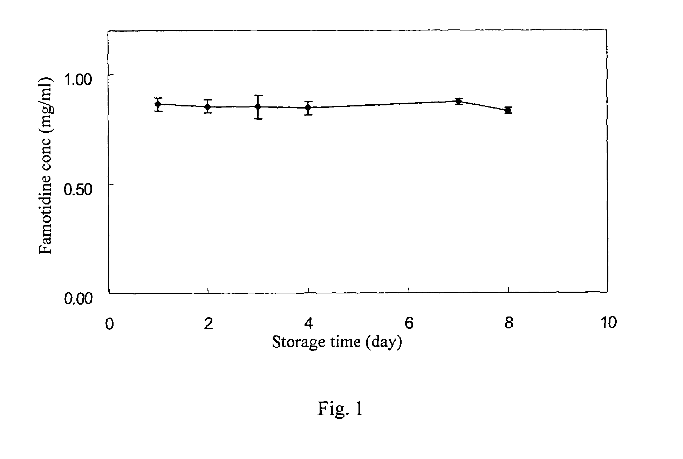

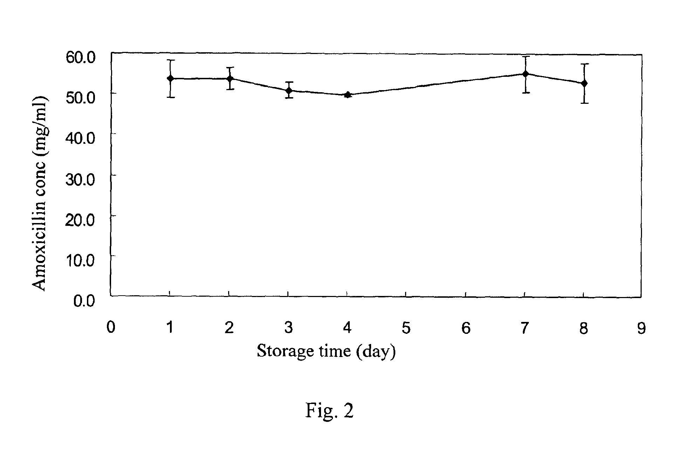

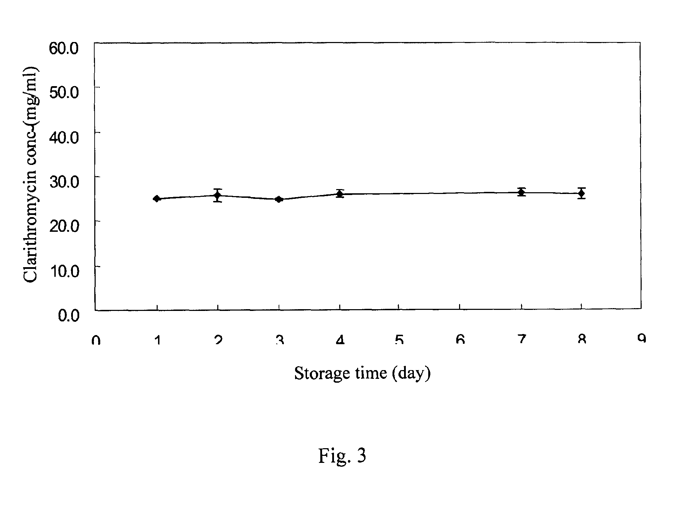

In a suspension type pharmaceutical composition, the pharmaceutical composition is mixed with a defined amount of water to form a suspension liquid and consumed in a proper step dose; the rest would be stored at 4°C for use in the next couple of days. The storage stability of the pharmaceutical compositions for the eradication of *Helicobacter pylori* in the suspension form was analyzed by adding 20 ml of water to 2.024 g of famotidine / amoxicillin/ clarithromycin suspension, and stored at 4°C in the dark for 8 days. The stabilities of famotidine, amoxicillin, and clarithromycin were shown in Fig. 1 to Fig. 3.

Referring to Fig. 1 to Fig. 3, the result of stabilities of famotidine, amoxicillin, and clarithromycin. The concentration of famotidine, amoxicillin, and clarithromycin showed no significant difference between day 1 and day 8, which indicated good stabilities for these antibiotics. In another words, the pharmaceutically acceptable carriers used in the preparation process of the pharmaceutical composition in the present invention provided a segregation function for β-lactam antibiotic, macrolide antibiotic and PPI, which would not contact each other after mixing, and thus provided the stability.

### Example 5 Bio-availability analysis of the pharmaceutical composition for the eradication of Helicobacter pylori in the effervescent tablet form

This experiment was to test the bioavailability of the pharmaceutical composition in animals, which is the blood concentration of the pharmaceutical composition in the effervescent tablet form after ingested by animals. Adult beagle dogs from 1 to 3 years of age and 10-12 kg in body weight were studied.

The effervescent tablet including 500 mg of amoxicillin, 250 mg of claritharmycin, and 20 mg of omeprazole was dissolved in 15 ml of water and supplied for one beagle dog in the experiment. Other commercial medications including 500 mg of amoxicillin (capsule), 250 mg of claritharmycin (tablet), and 20 mg of omeprazole (capsule) were used in the control group. The blood samples were taken and determined the concentration of each medication at 0, 0.33, 0.66, 1, 1.5, 2, 2.5, 3, 4, 6, 8, 10 and 24 after taking the medications.

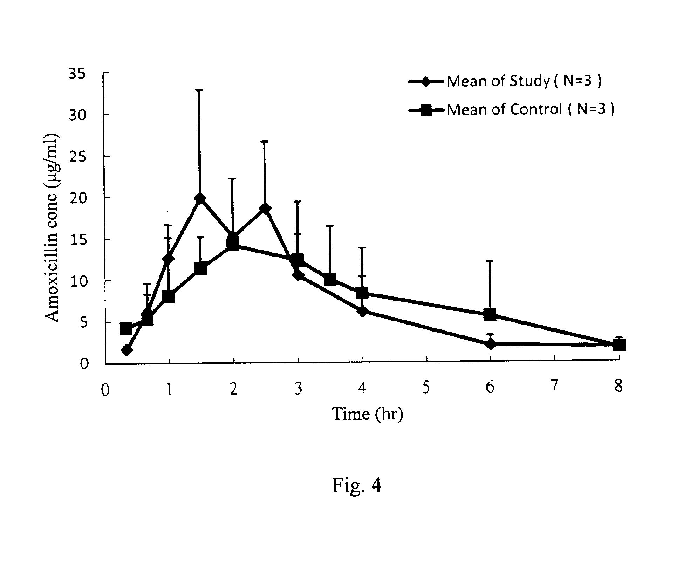

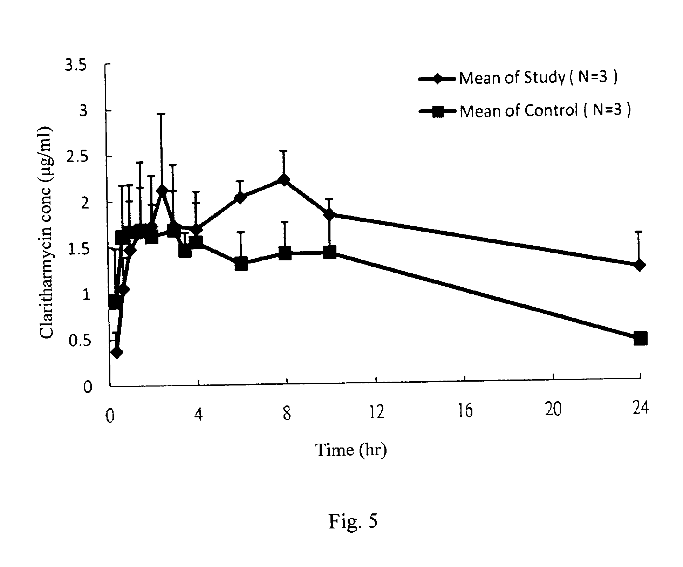

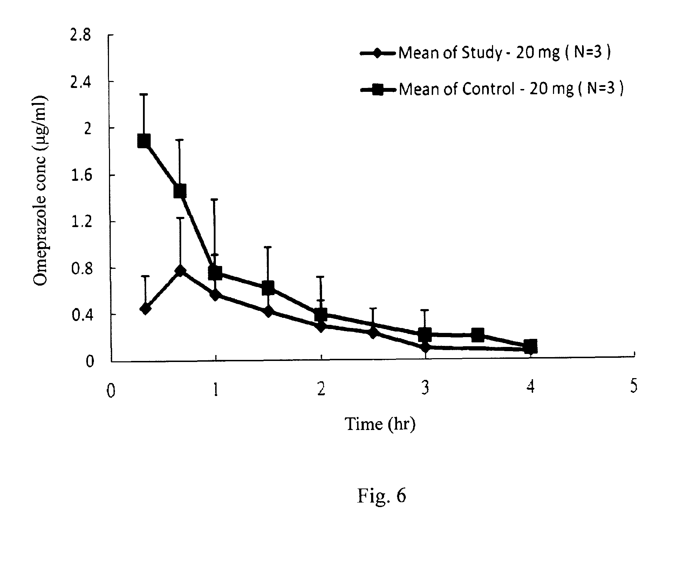

Fig. 4 to Fig. 6 showed the blood concentrations of amoxicillin, claritharmycin, and omeprazole in dogs. The released concentrations of amoxicillin, claritharmycin, and omeprazole from the pharmaceutical composition in the present invention in dogs were similar or even superior to those from the commercial capsules and tablets. The supplemented alkaline substances in the effervescent tablet according to the present invention could increase the pH in the stomach to a more alkaline condition to protect antibiotics or PPI from degradation due to strong acidic environment. As a result the pharmaceutical composition could work in the stomach to further increase the bioavailability.

The pharmaceutical compositions for the eradication of *Helicobacter pylori* in the forms of effervescent tablet, suspension or powder are produced in a simple way to have a similar or even better bioavailability than those from the commercial capsules and tablets. The pharmaceutical compositions have advantages such as good stability, fast disintegrating, rapid absorption, easy to swallow liquid formula, tolerant to gastrointestinal tract, easy to carry, flavor changeable (enhance flavor, easy for swallowing), applicable in large scale of medication, better absorption stability and more predictable on the outcome of therapy. In addition, the supplement of alkaline substance can increase the pH in the stomach to a more alkaline condition to protect antibiotics such as clarithromycin and amoxicillin from gastric acid degradation, to further eradicate *Helicobacter pylori* effectively.

On the other hand, the method for preparing a pharmaceutical composition for the eradication of *Helicobacter pylori* in the forms of effervescent tablet, suspension or powder according the present invention would not interact with each other and have stability because the compounds are mixed properly in order for preparation and supplemented with proper excipients. This preparation method is not limited to prepare pharmaceutical compositions for the eradication of *Helicobacter pylori* in the forms of effervescent tablet, suspension or powder according to the present invention, but also other pharmaceutical compositions for the eradication of *Helicobacter pylori.*

These above examples should not, however, be considered to limit the scope of the invention, it is contemplated that modifications will readily occur to those skilled in the art, which modifications will be within the spirit of the invention and the scope of the appended claims.

## Claims

1. A pharmaceutical composition for the eradication of *Helicobacter pylori,* comprising an effective dose of β-lactam antibiotic, an effective dose of macrolide antibiotic, an effective dose of antacid and a pharmaceutical acceptable carrier, wherein the pharmaceutical composition is present in the form of powder, suspension or effervescent tablet.

2. The pharmaceutical composition according to claim 1, wherein the β-lactam antibiotic is penicillin, phenoxypenicillin, oxacillin, cloxacillin, dicloxacillin, flucloxacillin, nafcillin, ampicillin, bacampicillin, amoxicillin, carbenicillin, ticarcillin, furbucillin, piperacillin, cefalotin, cefaloridin, cefalexin, cefazolin, cefradine, cefadroxil, cefamandole, cefuroxime, cefoxitin, cefotaxime, cefoperazone, ceftriaxone, ceftazidime, latamoxef, cefepime, cefpirome, cefclidin, aztreouam, imipenem, or meropenem.

3. The pharmaceutical composition according to claim 1, wherein the macrolide antibiotic is erythromycin, spiramycin, kitasamycin, midecamycin, jasamycin, roxithromycin, clarithromycin, azithromycin, tetracycline, oxytetracycline, demeclocycline, chlortetracycline, doxycycline, lymecycline, meclocycline, methacycline, minocycline, or rolitetracycline.

4. The pharmaceutical composition according to claim 1, wherein the antacids is a H₂ blocker or a proton pump inhibitor (PPI).

5. The pharmaceutical composition according to claim 4, wherein an effective dose of alkaline substance is added to increase the pH of the stomach when the antacid PPI is used.

6. The pharmaceutical composition according to claim 5, wherein the PPI is omeprazole, lansoprazole, esomeprazole, pantoprazole, tenatoprazole, rabeprazole, or an enantiomer, isomer, free base, salt, or mixture thereof.

7. The pharmaceutical composition according to claim 4, wherein the H₂ blocker is cimetidine, ranitidine, famotidine, nizatidine, or an enantiomer, isomer, free base, salt, or mixture thereof.

8. The pharmaceutical composition according to claim 5, wherein the alkaline substance is carbonate or bicarbonate.

9. The pharmaceutical composition according to claim 1, wherein the pharmaceutical composition further comprises one or more pharmaceutically acceptable carriers selected from the group consisting of excipients, flavor enhancers, effervescent agents, glidants, anti-bacterial agents, suspending agents, disintegrants, lubricants, fillers, binders, diluents, absorption enhancers and mixtures thereof.

10. A method for preparing a pharmaceutical composition for the eradication of *Helicobacter pylori* according to any of claims 1 to 9, comprising the steps:
(1) mixing an effective dose of β-lactam antibiotic and a pharmaceutical acceptable carrier to form a first mixture;
(2) mixing an effective dose of macrolide antibiotic and a pharmaceutical acceptable carrier to form a second mixture;
(3) mixing the first mixture and the second mixture with an antacid to form a third mixture; and
(4) mixing of the third mixture with a glidant, an anti-bacterial agent, a lubricants, and a suspending agent to form the pharmaceutical composition in powder form or suspension form.

11. A method for preparing a pharmaceutical composition for the eradication of *Helicobacter pylori* according to any of claims 1 to 9, comprising the steps:
(1) mixing a antacid, a suspending agent, an effective dose of macrolide antibiotic and a pharmaceutical acceptable carrier to form a first mixture;
(2) mixing an effective dose of β-lactam antibiotic and a pharmaceutical acceptable carrier including a lubricant to form a second mixture; and
(3) mixing the first mixture and the second mixture evenly to form the pharmaceutical composition in powder form or suspension form.

12. A method for preparing a pharmaceutical composition for the eradication of *Helicobacter pylori,* according to any of claims 1 to 9, comprising the steps:
(1) mixing an effective dose of β-lactam antibiotic, an effective dose of macrolide antibiotic , and a pharmaceutical acceptable carrier to form a first mixture;
(2) mixing an effective dose of an antacid, an anti-bacterial agent and a pharmaceutical acceptable carrier to form a second mixture;
(3) mixing the first mixture and the second mixture evenly with a foaming agent to formulate; and
(4) incorporating a glidant for compression formulation to produce the pharmaceutical composition in effervescent tablet form.
